Europäisches Patentamt

European Patent Office

Office européen des brevets

(11) Veröffentlichungsnummer: **0 046 533**
**B1**

(12) **EUROPÄISCHE PATENTSCHRIFT**

(45) Veröffentlichungstag der Patentschrift:
21.11.84

(21) Anmeldenummer: 81106211.6

(22) Anmeldetag: 08.08.81

(51) Int. Cl.³: **A 61 L 15/00, A 61 L 9/01**

(54) **Sanitäre Hygienemittel, die der Aufnahme Harn und Blut enthaltender Sekrete dienen, mit geruchsverhindernden Eigenschaften.**

(30) Priorität: **16.08.80 DE 3030920**

(43) Veröffentlichungstag der Anmeldung:
**03.03.82 Patentblatt 82/9**

(45) Bekanntmachung des Hinweises auf die Patenterteilung:
**21.11.84 Patentblatt 84/47**

(84) Benannte Vertragsstaaten:
**AT BE CH DE FR GB IT LI LU NL SE**

(56) Entgegenhaltungen:
**DE - A - 2 418 338**
**DE - A - 2 736 770**
**US - A - 3 707 148**
**US - A - 3 794 034**

*Die Akte enthält technische Angaben, die nach dem Eingang der Anmeldung eingereicht wurden und die nicht in dieser Patentschrift enthalten sind.*

(73) Patentinhaber: **Henkel Kommanditgesellschaft auf Aktien, Postfach 1100 Henkelstrasse 67, D-4000 Düsseldorf-Holthausen (DE)**

(72) Erfinder: **Schneider, Werner, Dr., Uerdinger Strasse 467, D-4150 Krefeld-Bockum (DE)**
Erfinder: **Sustmann, Scarlet, Dr., Am Roggenkamp 33, D-4400 Münster (DE)**

BUNDESDRUCKEREI BERLIN

**Beschreibung**

Gegenstand der Erfindung sind sanitäre Hygienemittel zur Aufnahme Harn und Blut enthaltender Sekrete, die in der Absorptionsschicht als geruchsverhindernde Substanz Ester der Citronensäure und/oder Acetylcitronensäure, insbesondere deren Triester, mit aliphatischen oder alicyclischen Alkoholen mit 1 bis 6 Kohlenstoffatomen im Molekül enthalten.

Als den erfindungsgmäßen sanitären Hygienemitteln mit geruchsverhindernden Eigenschaften zugrundeliegende Vorlagen können alle bisher für diese Zwecke eingesetzen Produkte dienen wie Windeln, Slipeinlage, Damenbinden, Tampons. Das diesen sanitären Hygienemitteln zugrundeliegende absorbierende Material kann aus jedem Fasermaterial bestehen, das üblicherweise zur Herstellung derartiger Mittel verwendet wird, wie gesponnener Wolle, Baumwolle, Cellulose-Chemiefasern, synthetischen Textilfasern, Vliesstoffen, aber auch aus quellfähigen Polymeren wie modifizierter Stärke, modifizierter Cellulose oder saugfähigen Kunststoffpartikeln, die gegebenenfalls zur besseren Handhabung auf Papier oder andere Laminate aufgeklebt oder in Netzen, Vliesstoff- bzw. Textilbahnen eingelagert sind.

Für eine wirksame Verhinderung der lokalen Zersetzung der in der absorbierenden Schicht aufgenommenen Sekrete ist es wichtig, daß eine möglichst gleichmäßige Durchdringung der Absorptionsschicht mit den Estern der Citronensäure und/oder Acetylcitronensäure erfolgt. Diese Forderung nach möglichst gleichmäßiger Durchdringung der Absorptionsschicht läßt sich mit den erfindungsgemäß einzusetzenden Produkten unschwierig erfüllen, da sie als Flüssigkeiten bei der Aufbringung in feinster Verteilung, z. B. als Nebeltröpfchen oder aus der Dampfphase keine Probleme aufwerfen. Ein weiterer Vorteil, der sich aus der flüssigen Beschaffenheit der erfindungsgemäß einzusetzenden Produkte ergibt, ist deren leichte Mischbarkeit mit gegebenenfalls zusätzlich aufzubringenden Duftstoffen.

Es ist bereits vorgeschlagen worden, sanitäre Hygienemittel mit Desinfektionsmitteln, medikamentösen Stoffen und Riechstoffen zu imprägnieren, um das Auftreten eines unangenehmen Geruchs soweit wie möglich zu verhindern. Zu diesem Zweck hat man Phenole, Quecksilberchlorid, schweflige Säure, in getrennter Anordnung Natriumacetat und Natriumbisulfat sowie unterschiedliche Bakterizide und Fungizide wie Chloramin, Actamer, Hexachlorophen, Bis-(tri-n-alkyl-zinn)sulfosalicylate, Phenylmercurisalze und ähnliche in die absorbierenden Schichten eingebracht. Derartige Zusätze unterdrücken in gewissem Maß die Entwicklung unangenehmer Gerüche, da sie die für die Entstehung der Gerüche verantwortlichen Mikroorganismen abtöten. Diese Unterdrückung unangenehmer Gerüche durch Abtötung für die Zersetzung von Blut und Harn verantwortlicher Mikroorganismen geht aber mit einer mehr oder weniger starken Schädigung der körpereigenen Haut- und Vaginalflora einher. Daneben treten bei dem Einsatz mancher Mittel Hautreizungen, gelegentliche Unverträglichkeiten, Lichtsensibilisierungen und toxische Nebenwirkungen unterschiedlicher Stärke auf, die einem verbreiteten Einsatz hindernd entgegenstehen. Man ist daher weiterhin bestrebt, Mittel aufzufinden, die die Entstehung unangenehmer Gerüche unterbinden, ohne daß sie die schädlichen Nebenwirkungen aufweisen.

Es wurde nun gefunden, daß sanitäre Hygienemittel zur Aufnahme Harn und Blut enthaltender Sekrete, die in der Absorptionsschicht als geruchsverhindernde Substanz Ester der Citronensäure und/oder Acetylcitronensäure, insbesondere deren Triester, mit aliphatischen oder alicyclischen Alkoholen mit 1 bis 6 Kohlenstoffatomen im Molekül enthalten, den gestellten Anforderungen weitestgehend gerecht werden.

Die erfindungsgemäß einzusetzenden Ester der Citronensäure lassen sich in bekannter Weise durch azeotrope Veresterung von Citronensäure mit dem jeweiligen Alkohol herstellen, wie dies z. B. für den Citronensäuretriethylester in der amerikanischen Patentschrift 20 76 111 (referiert im Chemischen Zentralblatt 1937, II, Seite 665) beschrieben ist. Die Herstellung der erfindungsgemäß einzusetzenden Acetylcitronensäureester kann durch Umsetzung der entsprechenden Citronensäureester mit Acetylchlorid, wie dies für den Acetylcitronensäuretriethylester von Wislicenus in Liebigs Annalen der Chemie Bd. 129, Seite 192, bzw. durch Umsetzung der entsprechenden Citronensäureester mit Essigsäureanhydrid, wie dies gleichfalls für den Acetylcitronensäuretriethylester in der amerikanischen Patentschrift 2 445 911 (referiert in Chemical Abstracts 1948, Spalte 8818) beschrieben ist, erfolgen.

Als zu veresternde Alkohole kommen z. B. Methanol, Ehtanol, Propanol, Isopropanol, Butanol-1, Butanol-2, 2-Methyl-propanol-1, 2-Methyl-propanol-2, 2-Methyl-butanol-1, 2-Methyl-butanol-4, n-Hexylalkohol, Ethylenglykol, Propylenglykol, Trimethylenglykol, Hexamethylenglykol, Glycerin, Erythrit, Sorbit und Cyclohexanol in Frage.

Unter den erfindungsgemäß einzusetzenden Estern der Citronensäure als auch der Acetylcitronensäure kommt den jeweiligen Triestern die größte Bedeutung zu und unter diesen zeichnet sich wiederum der Citronensäuretriethylester durch die stärkste geruchsverhindernde Wirkung als auch durch die beste anwendungstechnische Eignung aus. Der Citronensäuretriethylester ist als Lebensmittelzusatz zugelassen und somit als unschädlich einzustufen, selbst bei Überdosierungen können keine Hautreizungen oder andere Unverträglichkeitserscheinungen auftreten. Da der Citronensäuretriester nicht antibakteriell wirksam ist, kann durch seinen Einsatz auch keine aus medizinischer Sicht unerwünschte Störung der körpereigenen Haut- bzw. Vaginalflora eintreten.

Wie bereits vorstehend ausgeführt, ist eine möglichst gleichmäßige Durchdringung der absorbie-

# 0 046 533

renden Schicht mit den erfindungsgmäß einzusetzenden Mitteln die Voraussetzung dafür, daß keine lokalen Zersetzungserscheinungen und damit unerwünschte Geruchsbildung eintreten können. Um eine völlig homogene Imprägnierung zu erreichen, eignet sich am besten ein Verfahren, bei dem das Absorptionsmaterial oder die fertige Vorlage in eine evakuierte Kammer gebracht und nach Erzeugung eines Vakuums der Citronensäure- bzw. Acetylcitronensäureester bei erhöhter Temperatur bis zu seinem Sättigungsdruck verdampft wird. Das Material wird dabei gleichmäßig von dem Citronensäure- bzw. Acetylcitronensäureester durchdrungen und die Konzentrationen in den textilen oder papierartigen Materialien treten mit der Dampfphase ins Gleichgewicht. Die aufzubringende Menge an Citronensäure- bzw. Acetylcitronensäureester ist bei diesem Verfahren durch Verdampfungstemperatur und Vakuum exakt regulierbar. Für die Imprägnierung nach diesem Verfahren hat sich der Citronensäuretriethylester insgesamt als am besten geeignet erwiesen. In einem auf diese Weise völlig homogen imprägnierten sanitären Hygienemittel sind lokale Zersetzungen von Blut oder Harn enthaltenden Sekreten weitestgehend ausgeschlossen.

Da der Citronensäuretriethylester selbst chemisch relativ inert und geruchsneutral ist, läßt er sich ohne Beeinflussung der Geruchsqualität gut mit Parfümölen mischen. Dies ist von besonderem Vorteil, wenn eine gleichzeitige Parfümierung des sanitären Hygienemittels erwünscht ist.

Dabei sind der Citronensäuretriethylester und das Parfümöl ohne Schwierigkeit als Mischung applizierbar. Zu diesem Zweck wird das Absorptionsmaterial durch eine Kammer geführt, in der die Mischung aus Citronenäuretriethylester und Parfümöl fein zerstäubt wird, so daß infolge der geringen Tröpfchengröße die Absorptionsmaterialien gleichmäßig benetzt werden. Eine besonders feine Verteilung des Gemisches aus Citronensäuretriethylester und Parfümöl läßt sich durch Anlegen eines elektrischen Feldes an die Zerstäubungskammer erreichen.

Eine besonders vorteilhafte Ausführungsform einer Hygienevorlage (Slip, Binde) ergibt sich, wenn die mit dem Citronensäuretriethylester und gegebenenfalls Parfümöl imprägnierte, stark saugfähige Absorptionsschicht in der Mitte der Vorlage angeordnet (Abbildung Schicht 1), und von einer nicht imprägnierten Watteschicht oder Schicht aus gemahlener Zellwolle (Fluff) umgeben ist (Abb. Schicht 2). Daran anschließend nach außen hin befindet sich ein Hüllvlies (3), eine Wäscheschutzfolie aus Kunststoff oder wasserundurchlässigem Papier (4), ein Klebestreifen (5) und Siliconpapier (6). Die in der stark saugfähigen Absorptionsschicht befindlichen Polymeren mit hohem Saugvermögen, wie z. B. Stärkepfropfpolymerisate werden von dem Citronensäuretriethylester gut benetzt ohne zu quellen. Diese Benetzung des Polymeren durch den Citronensäuretriethylester verhindert jedoch in keiner Weise das Quellvermögen der Saugsubstanz. Der geschilderte Aufbau hat zwei Vorteile, zum einen wird durch die Lage der mit Citronensäuretriethylester benetzten hochsaugfähigen Absorptionsschicht eine Diffusion des Citronensäuretriethylesters zur Wäscheschutzfolie und zum Klebestreifen verhindert, zum anderen befindet sich der geruchsverhindernde Citronensäuretriethylester genau in dem Bereich, wo die Sekrete aufgenommen werden, da bei der geschilderten Ausführungsform die Sekretaufnahme fast ausschließlich in der imprägnierten hochsaugfähigen Schicht erfolgt, welche die Flüssigkeit anzieht und festhält. Die Unterbindung der Diffusion ist wünschenswert, da ansonsten eine Wellung der Folie und eine Klebkraftverminderung eintreten kann. Für die Konfektionierung wird die geschilderte Vorlage eingerollt oder eingeschlagen und anschließend einzeln cellophaniert oder mit einer opaken Folie umhüllt.


Nachweis der Hemmwirkung von Citronensäuretriethylester auf die Harnzersetzung


a) Geruchstest

4 g gemahlene Zellwolle (Fluff) wurden jeweils mit 0,5 g beziehungsweise 1,5 g Citronensäuretriethylester besprüht und gut durchmischt. Auf den so präparierten Fluff, sowie auf eine gleich Menge unbehandelten Fluffs wurden 25 ml frischer Urin gegeben. Es wurde 14 Stunden bei 37°C inkubiert, wobei das Gefäß durch einen luftdurchlässigen Wattestopfen verschlossen war. Anschließend wurden die Proben jeweils 5 verschiedenen Personen zum Geruchstest vorgelegt, wobei die Bezeichnung der Proben keinen Schluß auf eventuelle Zusätze erlaubte. Die Probe ohne Zusatz von Citronensäuretriethylester wurde in fünf unabhängigen Versuchsreihen jeweils von allen 5 Testpersonen spontan als die weitaus am unangenehmsten riechende identifiziert. Am stärksten wurde der Unterschied zwischen der Probe mit unpräpariertem Fluff und der Probe mit Fluff +1,5 g Citronensäuretriethylester empfunden. Die Konzentration von Citronensäuretriethylester betrug bei dieser Probe auf Urin bezogen 1,5%. Bei Annahme einer maximalen Menge von 3 ml Urin pro Slipeinlage (etwa des Fassungsvermögen einer Slipeinlage) führt demnach bereits eine Menge von 45 mg Citronensäuretriethylester pro Slipeinlage zu einem eindeutigen Ergebnis.


b) Hemmung der Alkalisierung

Bei der Zersetzung von Urin wird durch die Ureasetätigkeit Ammoniak freigesetzt, der zu einer

3

Verschiebung des ursprünglich sauren pH-Wertes zur basischen Seite führt. Zur Durchführung des Versuches wurden wiederum 4 g gemahlene Zellwolle (Fluff) bzw. 4 g Watte jeweils mit 0,5 g bzw. 1,5 g Citronensäuretriethylester besprüht und gut durchmischt. Auf das so präparierte Absorptionsmittel, sowie auf eine gleiche Menge unbehandeltes Absorptionsmittel wurden 25 ml frischer Urin gegeben. Diese Proben und eine Probe Urin ohne Zusatz von Absorptionsmittel wurden 14 Stunden bei 37°C inkubiert, wobei das Gefäß durch einen luftdurchlässigen Wattestopfen verschlossen war. Anschließend wurden die pH-Werte der einzelnen Proben gemessen und mit den vor der Inkubierung gemessenen pH-Werten verglichen. Die bei den Versuchen erhaltenen Werte sind nachstehender Tabelle zu entnehmen. In der Tabelle bedeutet U = Urin, F = Fluff, W = Watte, CAT = Citronensäuretriethylester, inkubiert über 14 Stunden = ink.

Tabelle 1

Hemmung der Alkalisierung

| Versuch | pH-Wert U frisch | U ink | U+F ink | U+W ink | U+F +0,5 g CAT ink | U+W +0,5 g CAT ink | U+F +1,5 g CAT ink | U+W +1,5 g CAT ink |
|---|---|---|---|---|---|---|---|---|
| 1 | 6,0 | 8,7 | 7,7 | — | 7,2 | — | 6,7 | — |
| 2 | 6,7 | 8,5 | 7,4 | — | 7,0 | — | 6,9 | — |
| 3 | 6,3 | 7,6 | — | 7,4 | — | 6,6 | — | 6,6 |

Wie der vorstehenden Tabelle I zu entnehmen ist, wurde in allen Fällen durch den Zusatz von Citronensäuretriethylester die Alkalisierung unterdrückt. Es wurden Differenzen von 0,5—1,0 pH-Einheiten beobachtet. Im Versuch 2 wurde der pH-Wert des frischen Urins durch Zugabe gealterten Urins heraufgesetzt. Aus der Tabelle ist ferner ersichtlich, daß bereits der Zusatz von Fluff bzw. Watte zu einer Reduzierung der Ureaseaktivität führt.

Wie den vorstehenden Versuchen zu entnehmen ist, läßt sich durch den Einsatz von Citronensäuretriethylester in den mit Urin versetzten sanitären Hygienemitteln eine Zersetzung und damit die Ausbildung unangenehmer Gerüche weitgehend unterbinden.

Entsprechend dem Geruchstest a) durchgeführte Versuche, wobei anstelle von Urin eine 1 : 1 verdünnte wäßrige Lösung von Schweineblut eingesetzt wurde, führten gleichfalls zu einer weitgehenden Unterdrückung unangenehmer Gerüche.

Aus der deutschen Auslegeschrift 24 18 338 war bereits die Verwendung von Estern der Citronensäure und/oder Acetylcitronensäure in wasserfreien kosmetischen Zubereitungen zur Unterdrückung von Körpergeruch bekannt. Es ließ sich aber aus der Tatsache, daß eine nicht antibakteriell wirksame Verbindung beim Aufbringen auf die Haut die Schweißzersetzung reduziert auf keinen Fall der Schluß ziehen, daß diese Verbindung als Zusatzstoff zu textilen und papierartigen Absorptionsmaterialien für Blut und Harn enthaltende Sekrete die Zersetzung dieser Sekrete und die Ausbildung unangenehmer Gerüche weitgehend unterbindet.

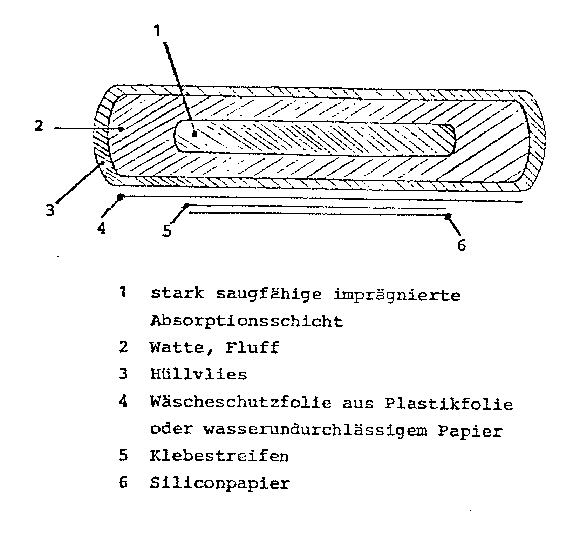

## Patentansprüche

1. Sanitäre Hygienemittel zur Aufnahme Harn und Blut enthaltender Sekrete, die in der Absorptionsschicht als geruchsverhindernde Substanz Ester der Citronensäure und/oder Acetylcitronensäure, insbesondere deren Triester, mit aliphatischen oder alicyclischen Alkoholen mit 1 bis 6 Kohlenstoffatomen im Molekül enthalten.

2. Sanitäre Hygienemittel nach Anspruch 1, dadurch gekennzeichnet, daß sie in der Absorptionsschicht Citronensäuretriethylester enthalten.

3. Verfahren zur Herstellung der sanitären Hygienenmittel nach Anspruch 1 und 2, dadurch gekennzeichnet, daß man das Absorptionsmaterial oder die fertige Vorlage in eine evakuierte Kammer bringt und nach Erzeugung eines Vakuums die Citronensäure- bzw. Acetylcitronensäureester bei erhöhter Temperatur bis zum Sättigungsdruck verdampft.

4. Verfahren zur Herstellung der sanitären Hygienemittel nach Anspruch 1 und 2, dadurch gekennzeichnet, daß man das Absorptionsmaterial durch eine Kammer führt, in der die Citronensäure- bzw. Acetylcitronensäureester gegebenenfalls im Gemisch mit Parfümöl fein zerstäubt werden.

5. Sanitäres Hygienemittel nach Anspruch 1 und 2 in Gestalt einer Hygienevorlage, bei der die mit dem Citronensäure- bzw. Acetylcitronensäureester imprägnierte, stark saugfähige Absorptionsschicht (1) in der Mitte der Vorlage angeordnet und von einer nicht imprägnierten Watteschicht oder Schicht aus gemahlener Zellwolle (2) umgeben ist.

## Claims

1. Sanitary hygiene articles for absorbing secreta containing urine and blood, said articles containing esters of citric acid and/or acetyl citric acid, more particularly triesters thereof, with aliphatic or alicyclic alcohols containing from 1 to 6 carbon atoms in the molecule as odor inhibitors in the absorption layer.

2. Sanitary hygiene articles as claimed in Claim 1, characterized in that they contain citric acid triethyl ester in the absorption layer.

3. A process for producing the sanitary hygiene articles claimed in Claims 1 and 2, characterized in that the absorption material or the actual pad is introduced into an evacuated chamber and, after a vacuum has been established, the citric acid or acetyl citric acid esters are evaporated at elevated temperature up to saturation pressure.

4. A process for producing the sanitary hygiene articles claimed in Claims 1 and 2, characterized in Claims 1 and 2, characterized in that the absorption material is passed through a chamber in which the citric acid or acityl citric acid esters are finely atomized, optionally in admixture with perfume oil.

5. A sanitary hygiene article as claimed in Claims 1 and 2 in the form of a hygiene pad in which the highly absorbent absorption layer (1) impregnated with the citric acid or acetyl citric acid ester is arranged in the middle of the pad and is surrounded by a non-impregnated layer of a nonwoven or of ground rayon stable (2).

## Revendications

1. Articles sanitaires d'hygiène conçus pour absorber les sécrétions contenant de l'urine et du sang, qui contiennent dans la couche absorbante, en tant que substance désodorisante, des esters de l'acide citrique et/ou de l'acide acétylcitrique, en pariculier leurs triesters, d'alcools aliphatiques ou alicycliques contenant 1 à 6 atomes de carbone dans la molécule.

2. Articles sanitaires d'hygiène selon la revendication 1 carctérisés en ce qu'ils contiennent du citrate de triéthyle dans la couche absorbante.

3. Procédé de préparation des articles sanitaires d'hygiène selon les revendications 1 et 2, caractérisé en ce que l'on introduit la matière absorbante ou l'article fini dans une chambre sous vide et après application d'un vide, on vaporise l'ester citrique ou acétylcitrique à chaud jusqu'à la pression de saturation.

4. Procédé de préparation des articles sanitaires d'hygiène selon des revendications 1 et 2, caractérisé en ce que l'on introduit la matière absorbante dans une chambre dans laquelle on atomise finement l'ester citrique ou acétylcitrique éventuellement en mélange avec un parfum.

5. Article sanitaire d'hygiène selon les revendications 1 et 2, sous la forme d'un article d'hygiène dans lequel la couche fortement absorbante (1) imprégnée d'ester citrique ou acétylcitrique est disposée au centre de l'article et enveloppée dans une couche d'ouate ou une couche de fibranne broyée (2) non imprégnée.

1   stark saugfähige imprägnierte
     Absorptionsschicht

2   Watte, Fluff

3   Hüllvlies

4   Wäscheschutzfolie aus Plastikfolie
     oder wasserundurchlässigem Papier

5   Klebestreifen

6   Siliconpapier

7